# EUROPEAN PATENT APPLICATION

(11) **EP 1 925 214 A1**
(43) Date of publication of application: **28.05.2008**
(21) Application number: 06024386.2
(22) Date of filing: 24.11.2006
(51) Int. Cl.: A23L 1/30, A61K 36/00, A23K 1/16

(54) **Dietary and pharmaceutical compositions containing carnosol and/or rosmanol and their uses**

(71) Applicant: DSM IP Assets B.V., 6411 TE Heerlen (NL); Nestec S.A., 1800 Vevey (CH)
(72) Inventor: The designation of the inventor has not yet been filed
(74) Representative: Steck, Melanie

(57) **Abstract**

The present invention refers to carnosol and rosmanol for use (as medicament) in the treatment of a disorder connected to impaired neurotransmission, as well as to dietary and pharmaceutical composition and their uses.

## Description

The present invention refers to carnosol and rosmanol for use as medicaments, especially for the treatment of disorders connected to impaired neurotransmission, as well as to dietary and pharmaceutical compositions containing one of them or both and their uses.

It is well known that impaired neurotransmission, e.g. low neurotransmitter levels, is connected to mental diseases such as depression and generalized anxiety disorders (GAD), and increased susceptibility to stress.

Compounds that increase neurotransmitter levels in the brain and thus enhance their transmission, exhibit therefore antidepressant properties as well as beneficial effects on a variety of other mental disorders ("Neurotransmitters, drugs and brain function" R.A. Webster (ed), John Wiley & Sons, New York, 2001, p. 187-211, 289-452, 477-498). The main neurotransmitters are serotonin, dopamine, noradrenaline (= norepinephrine), acetylcholine, glutamate, gamma-amino-butyric acid, as well as neuropeptides. Increase in neurotransmission is achieved by increasing the concentration of the synaptic cleft thus making it available for increased or prolonged neurotransmission through inhibition of re-uptake into the pre-synaptic nerve end, or by preventing neurotransmitter catabolism by inhibition of degrading enzymes such as monoaminooxidase A and B.

Tricyclic antidepressant compounds (TCAs) such as imipramine, amitriptyline, and clomipramine e-g, inhibit the re-uptake of serotonin and noradrenaline, They are widely regarded as among the most effective antidepressants available, but they have a number of disadvantages because they interact with a number of brain receptors, e.g, with cholinergic receptors. Most importantly, TCAs are not safe when taken in overdose, frequently showing acute cardiotoxicity.

Another class of antidepressant drugs are the so-called SSRIs (selective serotonin re-uptake inhibitors) including fluoxetine, patoxetine, sertraline, citalopram, fluvoxamine that block the serotonin transporter (SERT), a high affinity sodium chloride-dependent neurotransmitter transporter that terminates serotonergic neurotransmission by uptake of serotonin. They have been proven as effective in the treatment of depression and anxiety, and are usually better tolerated than TCAs. These medications are typically started at low dosages and may be increased until they reach a therapeutic level. A common side effect is nausea Other possible side effects include decreased appetite, dry mouth, sweating, infection, constipation, yawn, tremor, sleepiness and sexual dysfunction.

In addition, compounds that prevent the catabolism of neurotransmitters more broadly by inhibiting the monoaminooxidases (MAOs) A and B exhibit antidepressant effects. MAOs catalyse the oxidation of amino group containing neurotransmitters such as serotonin, noradrenaline, and dopamine.

Furthermore, modulators of neurotransmission exert pleiotropic effects on mental and cognitive functions.

There is a need for compounds for the treatment or prevention of mental diseases and/or disorders which do not show the negative side effects of known antidepressants. Many patients are interested in alternative therapies which could minimize the side effects associated with high-dose of drugs and yield additive clinical benefits. Severe depression is a long lasting and recurring disease, which is usually poorly diagnosed. Furthermore many patients suffer from mild or middle severe depression, Thus, there is an increasing interest in the development of compounds as well as pharmaceutical and/or dietary compositions that may be used to treat mental diseases/disorders or to prevent the development of mental diseases/disorders such as depression and dysthymia in people at risk, to stabilize mood and achieve emotional balance.

In addition, patients often suffer either as a comorbidity to depression, or by itself from generalized anxiety syndrome (GAD). GAD is a highly prevalent anxiety condition and chronic illness in primary care (~10% of patients) (Wittchen, HU and Jacoby, F. European Neuropsychopharmacology 15 (2005) 357 - 376). Patients present themselves to their primary care physician with multiple physical symptoms. GAD is characterized by chronic tension, and anxious worrying and tension (> 6 months), which are disabling and uncontrollable, and accompanied by a characteristic hypervigilance syndrome (including restlessness, muscle tension, and sleep problems). If untreated, GAD runs a chronic, fluctuating course and tends to get more severe with age. GAD patients suffer from subsyndromal depression and contribute to the highest overall direct and indirect health economic burden of all anxiety and depressive disorder. Despite high GAD incidence, few sufferers are diagnosed, prescribed medication, or receive psychiatric referral-simple diagnostic tools to aid patient recognition and monitoring are needed. Regardless of specific diagnosis, physicians require effective GAD-symptom treatments. SSRIs suche as paroxetine are effective for GAD treatment [Stocchi et al., Journal of Clinical Psychiatry, Efficacy and tolerability of paroxetine for the long-term treatment of generalized anxiety disorder, 2003, 63(3), 250-258]. Also, systematic reviews and placebo-controlled RCTs (Randomized Clinical Trials) indicate that some SSRIs (escitalopram, paroxetine and sertraline), the SNRI (Selective Norepinephrine Reuptake Inhibitors) venlafaxine, some benzodiazepines (alprazolam and diazepam), the tricyclic imipramine, and the 5-HT1A partial agonist buspirone are all efficacious in acute treatment. In general, the effect of treatment is often moderate and symptoms reappear when the treatment period is discontinued. Therefore, a continuous long-term treatment or prevention with compounds which have less side effects as SSRIs and can be taken over long time periods might by favourable over drug treatment.

Mood disorders and occupational stress also lead to consecutive sleep disorders, insomnia, low sleep quality, disturbances in circadian rhythms. These conditions are often chronic and can persist over long time. Also, deregulation of circadian rhythms induced by long-term flights (jet-lag) as well as by shift-working can cause similar symptoms and distress. Therefore, treatment with dietary supplementation to alleviate and prevent symptoms associated with the sleep disorders, such as impairment of cognitive function and memory, mental and physical fatigue, dreaminess, is warranted to improve the overall quality of life and benefiting vital energy of a person in need thereof.

According to the present invention this demand is met with carnosol and rosmanol, Thus, in one aspect the invention relates to carnosol and rosmanol and to any mixture of them for use as medicament for the treatment of a disorder connected to impaired neurotransmission.
In another aspect, the invention relates to the use of camosol and of rosmanol and of any mixture of them for the manufacture of a composition for the treatment of a disorder connected to impaired neurotransmission, particularly for the manufacture of an antidepressant, a mood/vitality improver, a stress reliever, a condition improver, a reducer of anxiety, a reducer of obsessive-compulsive behaviour, a relaxant, a sleep improver and/or a insomnia alleviator.

In still another aspect, the invention relates to a dietary composition containing at least either carnosol or rosmanol as well as to a pharmaceutical composition containing at least either camosol or rosmanol and a conventional pharmaceutical carrier.

Further, the invention relates to a method for the treatment of a disorder connected to impaired neurotransmission in animals including humans, said method comprising administering an effective dose of carnosol and/or rosmanol to animals including humans which are in need thereof.

Animals in the context of the present invention include humans and encompass mammals, fish and birds. Preferred "animals" are humans, pet animals and farm animals.

Examples for pet animals are dogs, cats, birds, toy fish, guinea pigs, (jack) rabbits, hares and ferrets. Examples for farm animals are fish, pigs, horses, ruminants (cattle, sheep and goat) and poultry.

The structures of carnosol (compound of formula I) and rosmanol (compound of formular II) are shown in Fig. I.

More preferred are (*4aR,9S, 10aS*)-carnosol and (*4aR, 9S, 10aS*)-rosmanol.

The term "rosmanol"/"carnosol" also encompasses any material or extract of a plant containing it or them in an amount of at least 30 weight-% (i.e. from 30 to 100 weight-%), preferably in an amount of at least 50 weight-% (i.e. from 50 to 100 weight-%), more preferably in an amount of at least 70 weight-% (i.e. from 70 to 100 weight-%), most preferably in an amount of at least 90 weight-% (i.e. from 90 to 100 weight-%), based on the total weight of the plant material or extract. The terms "material of a plant" and "plant material" used in the context of the present invention mean any part of a plant.

"Carnosol" means the racemic mixture as well as pure (*4aR,9S,10aS*)-carnosol or pure *(4aS, 9R, 10aR*)-carnosol or any mixture or diastereoisomer of them. Carnosol can be isolated from plants like the following, but not limited to *sage, Greek sage and rosemary.* Therefore, any material or extract of these plants or any other plant material or extract containing carnosol in an amount of at least 30 weight-% (i.e. from 30 to 100 weight-%), preferably in an amount of at least 50 weight-% (i.e. from 50 to 100 weight-%), more preferably in an amount of at least 70 weight-% (i.e. from 70 to 100 weight-%), most preferably in an amount of at least 90 weight-% (i.e. from 90 to 100 weight-%), based on the total weight of the plant material or extract, is also encompassed by this expression. "Carnosol" means both "natural" (isolated) and "synthetic" (manufactured) carnosol.

Carnosol's synthesis is described in several articles, i.e. in Tetrahedron 2003, 59(18), 3297-3305.

"Rosmanol" means the racemic mixture as well as pure (*4aR,9S, 10aS*)-rosmanol or pure (*4aS,9R,10aR*)-rosmanol or any mixture or diastereoisomer of them. Rosmanol can be isolated from plants like the following, but not limited to sage, magoram, rosemary, thyme, *Salivia sp., Lepechinia sp.* Therefore, any material or extract of these plants or any other plant material or extract containing rosmanol in an amount of at least 30 weight-% (i.e. from 3 0 to 100 weight-%), preferably in an amount of at least 50 weight-% (i.e. from 50 to 100 weight-%), more preferably in an amount of at least 70 weight-% (i.e. from 70 to 100 weight-%), most preferably in an amount of at least 90 weight-% (i.e. from 90 to 100 weight-%), based on the total weight of the plant material or extract, is also encompassed by this expression. "Rosmanol" means both "natural" (isolated) and "synthetic" (manufactured) rosmanol.

Rosmanol's synthesis is described e.g. in Journal of Natural Products 2002, 65(7), 986-989.

Beside the (pure) compounds carnosol and rosmanol especially preferred are plant materials and plant extracts containing at least 30 weight-% (i.e. from 30 to 100 weight-%), preferably at least 50 weight-% (i.e. from 50 to 100 weight-%), more preferably at least 70 weight-% (i.e. from 70 to 100 weight-%), most preferably at least 90 weight-% (i.e. from 90 to 100 weight-%), of these compounds, based on the total weight of the plant material/extract.

According to the present invention not only carnosol and rosmanol themselves but also plant materials and extracts containing them in an amount of at least 30 weight-% (i.e. from 30 to 100 weight-%), preferably in an amount of at least 50 weight-% (i.e. from 50 to 100 weight-%), more preferably in an amount of at least 70 weight-% (i.e. from 70 to 100 weight-%), most preferably in an amount of at least 90 weight-% (i.e. from 90 to 100 weight-%), based on the total weight of the plant material or extract, as well as dietary and pharmaceutical compositions containing them can be used as medicament, especially for the treatment of a disorder connected to impaired neurotransmission.

The dietary compositions according to the present invention may further contain protective hydrocolloids, binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellyfying agents, gel forming agents, antioxidants and antimicrobials.

The term "dietary compositions" comprises any type of (fortified) food/feed and beverages including also clinical nutrition, and also dietary supplements.

Beside a pharmaceutically acceptable carrier and at least either carnosol or rosmanol, the pharmaceutical compositions according to the present invention may further contain conventional pharmaceutical additives and adjuvants, excipients or diluents, flavoring agents, preservatives, stabilizers, emulsifying agents, buffers, lubricants, colorants, wetting agents, fillers, and the like. The carrier material can be organic or inorganic inert carrier material suitable for oral/parenteral/injectable administration.

The dietary and pharmaceutical compositions according to the present invention may be in any galenic form that is suitable for administrating to the animal body including the human body, especially in any form that is conventional for oral administration, e.g. in solid form such as (additives/supplements for) food or feed, food or feed premix, fortified food or feed, tablets, pills, granules, dragées, capsules, and effervescent formulations such as powders and tablets, or in liquid form such as solutions, emulsions or suspensions as e.g. beverages, pastes and oily suspensions. The pastes may be filled into hard or soft shell capsules. Examples for other application forms are forms for transdermal, parenteral or injectable administration. The dietary and pharmaceutical compositions may be in the form of controlled (delayed) release formulations.

Examples for fortified food are cereal bars, bakery items such as cakes and cookies.

Beverages encompass non-alcoholic and alcoholic drinks as well as liquid preparations to be added to drinking water and liquid food. Non-alcoholic drinks are e.g. soft drinks, sport drinks, fruit juices, lemonades, near-water drinks (i.e. water based drinks with a low calorie content), teas and milk based drinks. Liquid food are e.g. soups and dairy products (e.g. muesli drinks).

Carnosol and/or rosmanol can be used for the manufacture of compositions/medicaments for the treatment of a disorder connected to impaired neurotransmission.

In the context of this invention "treatment" also encompasses co-treatment as well as prevention. "Prevention" can be the prevention of the first occurrence (primary prevention) or the prevention of a reoccurence (secondary prevention).

Thus, the present invention is also directed to a method for the prevention of a disorder connected to impaired neurotransmission in animals including humans, said method comprising administering an effective dose of carnosol or rosmanol or any mixture of them to animals including humans which are in need thereof. In this regard an effective dose of carnosol and/or rosmanol, may especially be used for maintaining the mental well-being, for maintaining a balanced cognitive function, for helping to reduce the risk of mood swings, for helping to retain a positive mood and for supporting cognitive wellness, and for helping to maintain a good sleep quality.

In the context of this invention the term "disorder" also encompasses diseases.

Medicaments/Compositions for the treatment of disorders connected to impaired neurotransmission encompass antidepressants, mood/vitality improvers, stress relievers, condition improvers, anxiety reducers and obsessive-compulsive behaviour reducers, relaxants, sleep improvers and/or insomnia alleviators. They all improve, enhance and support the physiological neurotransmission, especially in the central nervous system, and therefore alleviate mental malfunction.

Antidepressants are medicaments/compositions for treating mental, behavioural and emotional/affective, neurotic, neurodegenerative, eating and stress related disorders such as e.g. unipolar depression, bipolar depression, acute depression, chronic depression, subchronic depression, dysthymia, postpartum depression, premenstrual dysphoria/syndrom (PMS), climacteric depressive symptoms, aggression, attention deficit disorders (ADS), social anxiety disorders, seasonal affective disorders, anxiety (disorders) such as generalized anxiety disorder (GAD), fibromyalgia syndrome, post-traumatic stress disorders, panic disorders, obsessive compulsive disorders, restless leg syndrome, nervousness, migraine/primary headaches and pain in general, emesis, bulimia, anorexia nervosa, binge eating disorder, gastrointestinal disorders, burn out syndrome, and irritability.

Antidepressants can also be used for (the manufacture of compositions for) primary and secondary prevention and/or the treatment of neurocognitive impairment. Furthermore they are also effective in the treatment of depressive symptoms or other symptoms related to disturbed neurotransmission occurring as comorbidity in chronic diseases such as cardiovascular diseases, strokes, cancer, Alzheimer disease, Parkinson disease, and others.

Carnosol and/or rosmanol as well as (mixtures of) plant materials and plant extracts containing them (especially in an amount of at least 30 weight-%, preferably in an amount of at least 50 weight-%, more preferably in an amount of from 70 to 90 weight-%, most preferably in an amount of at least 90 weight-%, based on the total weight of the plant material or extract), and dietary/pharmaceutical compositions containing them are thus suitable for the treatment of animals including humans.

Especially pet animals and farm animals can be in conditions in need of enhanced or improved neurotransmission. Such conditions e.g. occur after capture or transport or with housing, when the animals develop analogous disorders and are distressed or aggressive, or display stereotypic behaviour, or anxiety and obsessive-compulsive behaviour.

Thus, carnosol or rosmanol or any mixture of them can be used in general as antidepressants for animals including humans, preferably for humans, pet animals and farm animals.

In a further embodiment of the present invention carnosol and/or rosmanol find use as mood improver in general as well as for the manufacture of compositions for such use (plant materials/extracts containing them in an amount of at least 70 weight-%, preferably in an amount of at least 90 weight-%, based on the total weight of the plant material or extract; dietary/pharmaceutical compositions). "Mood improver" or "emotional wellness booster" or "vitality improver" means that the mood of a person treated with it is enhanced, that the self esteem is increased and/or that negative thoughts and/or negative tension are/is reduced. It also means the emotions are balanced and/or that the general, especially the mental, well being and vitality is improved or maintained, as well as that the risk of mood swings is (helped to be) reduced and that the positive mood is (helped to be) retained.

Carnosol or rosmanol or any mixture of them can also be used in general as anxiety reducer and/or obsessive-compulsive behaviour reducer for animals including humans, preferably for humans, pet animals and farm animals.

Anxiety reducer means that chronic tension and anxious worrying and tension are alleviated and relieved. Hypervigilance syndrome, including restlessness, muscle tension, and sleep problems are relieved. Social and other phobias are resolved. In general, the social environment is experienced less threatening. The person is emotionally relaxed, experiences comfort and enjoys company and contact to other people.

"Relaxant" or "sleep improver" or "insomnia alleviator" means improving sleep onset and helping a person to easily enter sleep, to maintain an undisrupted sleep over the night. It also means to correct circadian rhythm associated sleep disturbances due to jet-lag or shift work, and to prevent and abolish the symptoms associated with sleeplessness, i.e. impairment of cognitive function and memory, mental and physical fatigue, dreaminess, and improve overall quality of life and vital energy,

Moreover, carnosol or rosmanol as well as compositions comprising an effective dose of it/them are useful for the treatment, prevention and the alleviation of stress related symptoms, for the treatment, prevention and alleviation of symptoms related to working overload, exhaustion and/or burn out, for the increase of the resistance or tolerance to stress and/or to favor and facilitate the relaxation in normal healthy individuals i.e. such compositions have an effect as "stress reliever".

Furthermore, carnosol and/or rosmanol as well as compositions comprising an effective dose of them are useful for the treatment, prevention and alleviation of anxiety and obsessive-compulsive behaviour in humans and animals.

A further embodiment of the present invention relates to the use of carnosol and/or rosmanol and to the use of compositions containing it/them (plant materials/extracts containing it/them in an amount of at least 70 weight-%, preferably in an amount of at least 90 weight-%, based on the total weight of the plant material or extract; dietary/pharmaceutical compositions) as "condition improver", i.e, as means to reduce irritability and tiredness, to reduce or prevent or alleviate physical and mental fatigue, and to increase energy in more general terms, especially to increase the brain energy production, in diseased or normal healthy individuals. Moreover for cognition improvement in general, and especially for maintenance or improvement of attention and concentration, of the memory and of the capacity for remembering, of the learning ability, of the language processing, of problem solving and of intellectual functioning; for improvement of the short-term memory; for increasing the mental alertness; for enhancing the mental vigilance; for reducing the mental fatigue; for supporting cognitive wellness, for maintaining balanced cognitive function, for the regulation of hunger and satiety as well as for the regulation of motor activity.

The present invention not only refers to carnosol and rosmanol and their compositions (i.e. (mixture of) plant extracts containing them in an amount of at least 70 weight-%, preferably in an amount of at least 90 weight-%, based on the total weight of the plant material or extract; dietary/pharmaceutical compositions containing them) for use as medicaments, especially for the treatment of disorders connected to impaired neurotransmission, but also for the methods for the treatment of such disorders themselves, as already mentioned above.

In an especially preferred embodiment of such method pet animals or farm animals whose disorders are associated with housing, capture or transport are treated and which may appear in form of anxiety, stereotypic or obsessive-compulsive behavior.

For humans a suitable daily dosage of carnosol or rosmanol or any mixture of them for the purposes of the present invention may be within the range of from 0.001 mg per kg body weight to about 20 mg per kg body weight per day. More preferred is a daily dosage of from about 0.01 to about 10 mg per kg body weight, and especially preferred is a daily dosage of from about 0,05 to 5.0 mg per kg body weight. The amount of a plant material or plant extract containing such either carnosol or rosmanol or both of them can be calculated accordingly.

In solid dosage unit preparations for humans, carnosol or rosmanol or any mixture of them, is suitably present in an amount in the range of from about 0.1 mg to about 1000 mg, preferably in the range of from about 1 mg to about 500 mg per dosage unit.

In dietary compositions, especially in food and beverages for humans, camosol or rosmanol or any mixture of them, is suitably present in an amount in the range of from about 0.0001 (1 mg/kg) to about 5 weight-% (50 g/kg), preferably from about 0.001 % (10 mg/kg) to about 1 weight-%, (10 g/kg) more preferably from about 0.01 (100 mg/kg) to about 0.5 weight-% (5 g/kg), based upon the total weight of the food or beverage.

In food and drinks in a preferred embodiment of the invention the amount of the tricyclic diterpene or the derivative thereof according to formulae I and II, with the definitions of R¹ to R¹⁰ and the preferences as given above is in the range of from 10 to 30 mg per serving, i.e. 120 mg per kg food or drink.

For animals excluding humans a suitable daily dosage of carnosol or rosmanol or any mixture of them, for the purposes of the present invention may be within the range of from 0.001 mg per kg body weight to about 1000 mg per kg body weight per day. More preferred is a daily dosage in the range of from about 0.1 mg to about 500 mg per kg body weight, and especially preferred is a daily dosage in the range of from about 1 mg to 100 mg per kg body weight.

The invention is illustrated further by the following examples.

### Examples

The Carnosol and Rosmanol used in the experiments were synthesized by chemists of DSM Nutritional Products Ltd., Kaiseraugst, Switzerland. All compounds were > 95% pure.

### Example 1: Serotonin uptake inhibition by carnosol and rosmanol.

HEK-293 cells stably expressing the human serotonin re-uptake transporter (hSERT) were obtained from R. Blakely, Vanderbilt University, USA, The cells were routinely grown in Dulbeco's Modified Eagles Medium (Bioconcept) containing 10% fetal calf serum, penicillin, streptomycin, L-glutamine and the antibiotic G418 and passaged by trypsinisation. On the day of assay, cells from 80% confluent flasks were harvested by gentle washing with warm phosphate buffered saline (PBS). Cells were then washed once by centrifugation and re-suspended in Krebs Ringers bicarbonate buffer (Sigma) supplemented with 35 µM pargyline, 2.2 mM CaCl₂, 1 mM ascorbic acid and 5 mM N-2-hydroxyethylpiperazine-N'-2-ethanesulfonic acid (buffer called "Hepes") at a concentration of 10,000 cells in 160 ul of buffer, and aliquoted into round bottomed polypropylene 96 well microtitre plates (Coming) at 10,000 cells per well. Serotonin uptake into the cells was determined by addition of radio-labeled (3H) serotonin (GE Healthcare) to a concentration of 20 nM, and incubation for 40 minutes at 37°C with gentle shaking. At the end of this time unincorporated label was removed by filtration though Unifilter 96 GF/B plates (Perkin Elmer) using a Tomtec Mach III M cell harvester. The incorporated serotonin retained on the plates was quantified by liquid scintillation counting using Microscint-40 / Topcount (Perkin Elmer).

The effect of carnosol and rosmanol on the serotonin uptake was determined by their inclusion in the assay at a range of concentrations between 0.03 and 100 µM for 10 minutes prior to and during the addition of(3H) serotonin. Serotonin uptake via the transporter was inhibited by each of them in a dose dependent manner. The calculated IC₅₀ values for inhibition of serotonin uptake by each of them are shown in Table 1.

**Table 1: Inhibition of serotonin uptake into transfected HEK-293 cells by carnosol and rosmanol**

| **Substance** | **IC₅₀ [µM] for Tritiated Serotonin Uptake** |
|---|---|
| Carnosol | 26.6 +/- 6.9 s.e.m. n = 2 |
| Rosmanol | 23.2 +/- 9.7 s.e.m. n = 2 |

If one experiment was carried out twice or more (n) the average value +/- the standard error in the mean (s.e.m.) is shown.

### Example 2: Monoaminooxidase inhibition by carnsol and rosmanol

The organic amines p-tyramine or benzylamine were used as substrates for the Monoamine oxidase A (MAO-A) and B (MAO-B) enzymes respectively. The H₂O₂ produced by this reaction was quantified by reaction with vanillic acid, catalysed by horse radish peroxidase (HRP).

The reactions were carried out in polystyrene microtitre plates. The MAO enzymes (final concentration 2 U/ml) were mixed with either p-tyramine (Sigma, final concentration 0.5 mM) or benzylamine (Sigma, final concentration 0.5 mM) as appropriate and the chromogenic solution (containing vanillic acid (Fluka), 4-aminoantipyrine (Fluka) and horse radish peroxidase (Sigma), final concentrations 0.25 mM, 0.125 mM and 1, U/ml respectively) in 0.2 M potassium phosphate buffer pH 7.6. The reactions were followed in a microtitre plate absorbance reader eg Spectramax M5 (Molceular Devices Corporation). Absorbance readings at 495 nm were taken every 15 seconds for 40 minutes and the initial reaction velocties calculated by linear regression using SOFTmaxPro (Molecular Devices Corporation).

The effect of carnosol and rosmanol, respectively on the monoamine oxidase enzymes was determined by their inclusion in the assay at a range of concentrations between 0.03 and 100 µM for 10 minutes prior to and during the incubation with substrate. To determine the effect of the compounds on the HRP catalyzed portion of the reaction, the MAO enzyme was replaced by H₂O₂ (Molecular Probes, final concentration 0.2 mM). The reactions containing MAO-A and MAO-B were both inhibited by each of them in a dose dependent manner, whilst the control reaction, was unaffected. The measured IC50 values for inhibition of monoamine oxidase activity by carnosol and rosmanol are shown in Table 2.

**Table 2: Inhibition of MAO-A and MAO-B by-carnosol and rosmanol**

| **Substance** | **IC50 [µM] for Inhibition of MAO-A** | **IC50 [µM] for Inhibition of MAO-B** |
|---|---|---|
| Carnosol | 4.0 +/- 0.1 n = 3 | 106.5 +/- 19.9 n = 2 |
| Rosmanol | 3.02 +/- 0.48 n = 3 | 73.3 +/- 11.3 n = 2 |

If one experiment was carried out twice or more (n) the average value +/- the standard error in the mean (s.e.m.) is shown.

### Example 3: Porsolt's swim test

The Forced Swim Test (FST) was first reported in 1977 for screening of antidepressant-like compounds in rats (PORSOLT, R.D., LE PICHON, M. & JALFRE, M. (1977). Depression: a new animal model sensitive to antidepressant treatments. Nature, 266, 730-732) and, later, modified for testing mice (PORSOLT, R,D., BERTIN, A. & JALFRE, M. (1977), Behavioural despair in mice: a primary screening test for antidepressants. Archives Intemationales de Pharmacadynamie, 229, 327-336.). "Behavioural despair" was demonstrated whereby, when forced to swim in a cylinder of water from which there is no escape, rats or mice will initially exhibit vigorous, escape-oriented, activity but eventually only make those minimal movements necessary to keep their heads above water. The test was shown to be sensitive to a range of drugs with known therapeutic activity against depression, some of which had not previously shown efficacy in the existing behavioural models (R.D. PORSOLT. et al., Nature 1977, 266, 730-732.).

### Carnosol

Mice were individually placed in a cylinder (Height = 24 cm; Diameter =13 cm) containing 10 cm water (22° C) from which they could not escape, The mice were placed in the water for 6 minutes and the duration of immobility during the last 4 minutes was measured. 15 mice were studied per group, The test was performed blind. Carnosol was evaluated at 3, 10, 30mg/kg body weight, administered i.p. 30 minutes before the test, and compared with a vehicle control group. Imipramine (32 mg/kg i.p,), administered under the same experimental conditions, was used as reference substance. Immobility and swimming time was assessed.

### Results

**Table 3: Effects of carnosol and imipramine in the FST in the mouse**

| Carnosol (mg/kg) i.p. -30 min | DURATION OF IMMOBILITY (s) | | |
|---|---|---|---|
| | mean ± s.e.m. | p value | % change from control |
| Vehicle | 169.1 ±8.4 | - | - |
| 3 | 125.4 ±10.5 ** | 0.0030 | -26% |
| 10 | 132,9 ±12.3 * | 0.0217 | -21% |
| 30 | 139.3 ±10.8 * | 0.0380 | -18% |
| IMIPRAMINE 32 mg/kg i.p. -30 min | 34.6 ±9.8 *** | <0.0001 | -80% |

Thus, all doses of carnosol tested in the FST significantly reduced immobility behavior.

### Example 4: Marble burying test

"Defensive burying" behaviour was demonstrated by rats burying noxious objects, such as drinking spouts filled with a unpleasant-tasting liquid (WILKIE, D.M., MACLENNAN, A.J. & PINEL, J.P.J. (1979). Rat defensive behavior: burying noxious food. Journal of the Experimental Analysis of Behavior, 31, 299-306.) or shock prods (PTNEL, J.P.J. & TREIT, D. (1978). Burying as a defensive response in rats. Journal of Comparative and Physiological Psychology, 92, 708-712.). The marble burying test was devised as a modification of such a test, Poling et al, (POLING, A., CLEARY, J. & MONAGHAN, M. (1981). Burying by rats in response to aversive and nonaversive stimuli. Journal of the Experimental Analysis of Behavior, 35, 31-44.) exposed rats to individual cages each containing 25 marbles, daily for 10 or 21 consecutive days, The number of marbles buried, on each day of the 10 day (d) period, or 24 hours (h) after the 21 d exposure, were counted. The authors reported that the burying of marbles was not determined by novelty, or due to any noxious stimuli.

Marble burying behaviour by mice is reported to be sensitive to a range of minor (e.g. diazepam) and major (e.g, haloperidol) tranquilisers (BROEKKAMP, C.L., RIJK, H.W., JOLY-GELOUIN, D. & LLOYD, K.L. (1986). Major tranquillizers can be distinguished from minor tranquillizers on the basis of effects on marble burying and swim-induced grooming in mice. European Journal of Pharmacology, 126,223-229.), in addition to SSRIs (e.g. fluvoxamine, fluoxetine, citalopram), tricyclic antidepressants (e.g. imipramine, desipramine) and selective noradrenaline uptake inhibitors (e.g. reboxetine), at doses which do not induce sedation, The model may reflect either anxiety-like- or obsessive-compulsive- behaviour (see DE BOER, S.F. & KOOLHAAS, J,M. (2003). Defensive burying in rodents: ethology, neurobiology and psychopharmacology. European Journal of Pharmacology, 463,145-161.).

The method applied here follows that described by Broekkamp et al. (European Journal of Pharmacology 1986, 126, 223-229.). Mice (n =15 per treatment group) were individually placed in transparent plastic cages (33 x 21 x 18 cm) with 5 cm of sawdust on the floor and 25 marbles (diameter 1 cm) grouped in the centre of the cage. A second, up-tumed, cage served as a lid. The number of marbles covered by sawdust (by at least two-thirds) was counted at the end of the 30-minute test period. Tests were performed by investigators blind to the drug treatment protocol,

Prior to testing, all test cages and marbles were "impregnated" by leaving 10 naive mice in each cage for 15 minutes.

Carnosol was evaluated at 3, 10 and 30 mg/kg, administered intraperitonally (i.p.) 30 minutes before the test, and compared with a vehicle control group. Fluoxetine (32 mg/kg), administered under the same experimental conditions, was used as a reference substance.

Data were analysed by comparing treated groups with vehicle control using unpaired Student's t-tests.

### Results

**Table 4: Effects of carnosol and fluoxetine in the marble burying test in the mouse**

| Carnosol (mg/kg) i.p. -30 min | NUMBER OF MARBLES COVERED BY SAWDUST | | |
|---|---|---|---|
| | mean ± s.e.m. | p value | % change from control |
| Vehicle | 20.7 ±1.2 | - | - |
| 3 | 18.3 ± 2.0 (NS) | 0.2966 | -12% |
| 10 | 18.7 ± 1.7 (NS) | 0.3356 | -10% |
| 30 | 12.2 ± 2.7 (**) | <0.0068 | -41% |
| FLUOXETINE 32 mg/kg i.p. -30 min | 1.6± 1.6 (***) | <0.0001 | -92% |

| | | | |
|---|---|---|---|
| Student's t test: NS = Not Significant; *** = p < 0.001 | | | |

The highest dose of carnosol tested (30 mg/kg) clearly and significantly reduced marble burying behaviour in a similar manner to the SSRI fluoxetine.

### Example 5: Effect of carnosol in the Four Plates Test

The method, which detects anxiolytic activity, follows that described by Aron et al. (Aron C., Simon P., Larousse C., Boissier J.R. Evaluation of a rapid technique for detecting minor tranquilizers. Neuropharmacology, 10, 459-469, 1971). Anxiolytics (benzodiazepines) increase the number of punished crossings.

Animals were placed individually in a white plastic enclosure containing a floor consisting of 4 metal plates connected to an electric shock generator (Apelex: type 011346). The mouse was left to explore freely for 15 seconds. Then, every time it crossed from one plate to another, it received a weak electric shock (2.5 mA, 1.5 s). The number of punished crossings was counted during a 1 minute test. 15 mice were studied per group. The test was performed blind.

Camosol was evaluated at 3 doses (200, 400, 600 mg/kg, administered p.o. 3 times at 24 hours, 5 hours and 60 minutes before the test, and compared with a vehicle control (corn oil) group.

Clobazam (32 mg/kg p.o.), administered 1 hour before the test, was used as reference substance. Mice in this group received additional administrations of vehicle at 24 hours and 5 hours before the test in order to maintain experimental blinding.

**Table 5: Effects of carnosol in the Four Plates Test in the mouse**

| TREATMENT (mg/kg) p.o.-24 h and -5 h | TREATMENT (mg/kg) p.o. -60 min | NUMBER OF PUNISHED CROSSINGS | | | | | |
|---|---|---|---|---|---|---|---|
| | | mean ± s.e.m. | | | | p value | % change from control |
| Vehicle | Vehicle | 2.8 | ± | 0.2 | | - | - |
| Carnosol (200) | carnosol (200) | 3.5 | ± | 0.3 | NS | 0.0713 | +25% |
| Carnosol(400) | carnosol(400) | 3.7 | ± | 0.3 | * | 0.0196 | +32% |
| Carnosol (600) | carnosol (600) | 3.1 | ± | 0.4 | NS | 0.4406 | +11% |
| Vehicle | Clobazam(32) | 6.1 | ± | 0.5 | *** | <0.0001 | +118% |

Carnosol (200, 400 and 600 mg/kg) globally increased the number of punished crossings, as compared with vehicle control (+25%, +32% and +11%, respectively), significantly so at 400 mg/kg (p < 0.05). These results suggest that carnosol has Weak but significant anxiolytic-like activity at 400 mg/kg p.o., but not at lower or higher doses, in the same test.

### Example 6: Preparation of a soft gelatin capsule

A soft gelatin capsule (500 mg) is prepared comprising the following ingredients:

| **Ingredient** | **Amount per Capsule** |
|---|---|
| Carnosol or Rosmanol | 200 mg |
| Lecithin | 50 mg |
| Soy bean oil | 250 mg |

Two capsules per day for 3 months may be administered to a human adult for the treatment of mild chronic dysthymia.

### Example 7: Preparation of a soft gelatin capsule

A soft gelatin capsule (600 mg) is prepared comprising the following ingredients:

| **Ingredient** | **Amount per Capsule** |
|---|---|
| Camosol | 100 mg |
| Rosmanol | 100 mg |
| Evening prim rose oil | 300 mg |
| Vitamin B₆ | 100 mg |

One capsule per day preferably at the second half of the menstrual cycle should be taken for 14 days for the treatment of premenstrual syndrome and premenstrual dysphoric disorder.

### Example 8: Preparation of a tablet

A 400 mg-tablet is prepared comprising the following ingredients:

| **Ingredient** | **Amount per tablet** |
|---|---|
| Carnosol or Rosmanol | 100 mg |
| Passion flower standardized extract | 150 mg |
| Green Tea Extract, e.g. TEAVIGO® from DSM Nutritional Products, Kalseraugst, Switzerland | 150 mg |

For general well being, energizing and stress alleviation, one tablet is taken twice daily for 3 months.

### Example 9: Preparation of an instant flavoured soft drink

| **Ingredient** | **Amount [g]** |
|---|---|
| Camosol or Rosmanol | 0.9 |
| Sucrose, fine powder | 922.7 |
| Ascorbic acid, fine powder | 2.0 |
| Citric acid anhydrous powder | 55.0 |
| Lemon flavour | 8.0 |
| Trisodium citrate anhydrous powder | 6.0 |
| Tricalciumphosphate | 5.0 |
| β-Carotene 1% CWS from DNP AG, Kaiseraugst, Switzerland | 0.4 |
| **Total amount** | **1000** |

All ingredients are blended and sieved through a 500 µm sieve. The resulting powder is put in an appropriate container and mixed on a turbular blender for at least 20 minutes, For preparing the drink, 125 g of the obtained mixed powder are taken and filled up with water to one liter of beverage.
The ready-to-drink soft drink contains ca. 30 mg camosol per serving (250 ml).
As a strenghtener and for general well being 2 servings per day (240ml) should be drunk.

### Example 10: Preparation of a fortified non baked cereal bar

| **Ingredient** | **Amount [g]** |
|---|---|
| Carnosol or Rosmanol | 0.95 |
| Sugar | 114.55 |
| Water | 54.0 |
| Salt | 1.5 |
| Glucose syrup | 130.0 |
| Invert sugar syrup | 95.0 |
| Sorbitol Syrup | 35.0 |
| Palmkernel fat | 60.0 |
| Baking fat | 40.0 |
| Lecithin | 1.5 |
| Hardenend palm-oil | 2.5 |
| Dried and cut apple | 63.0 |
| Cornflakes | 100,0 |
| Rice crispies | 120.0 |
| Wheat crispies | 90.0 |
| Roasted hazelnut | 40.0 |
| Skim milk powder | 45.0 |
| Apple flavour 74863-33 | 2.0 |
| Citric acid | 5.0 |
| **Total amount** | 1000 |

Carnosol or Rosmanol is premixed with skim milk powder and placed in a planetary bowl mixer. Cornflakes and rice crispies are added and the total is mixed gently. Then the dried and cut apples are added. In a first cooking pot sugar, water and salt are mixed in the amounts given above (solution 1). In a second cooking pot glucose, invert and sorbitol syrup are mixed in the amounts given above (solution 2). A mixture of baking fat, palm-kernel fat, lecithin and emulsifier is the fat phase. Solution 1 is heated to 110°C. Solution 2 is heated to 113°C and then cooled in a cold water bath. Afterwards solution 1 and 2 are combined. The fat phase is melted at 75°C in a water bath. The fat phase is added to the combined mixture of solution 1 and 2. Apple flavour and citric acid are added to the liquid sugar-fat mix. The liquid mass is added to the dry ingredients and mixed well in the planetary bowl mixer. The mass is put on a marble plate and rolled to the desired thickness. The mass is cooled down to room temperature and cut into pieces. The non baked cereal bar contains ca. 25 mg carnosol or rosmanol acid per serving (30 g). For general wellbeing and energizing 1-2 cereal bars should be eaten per day,

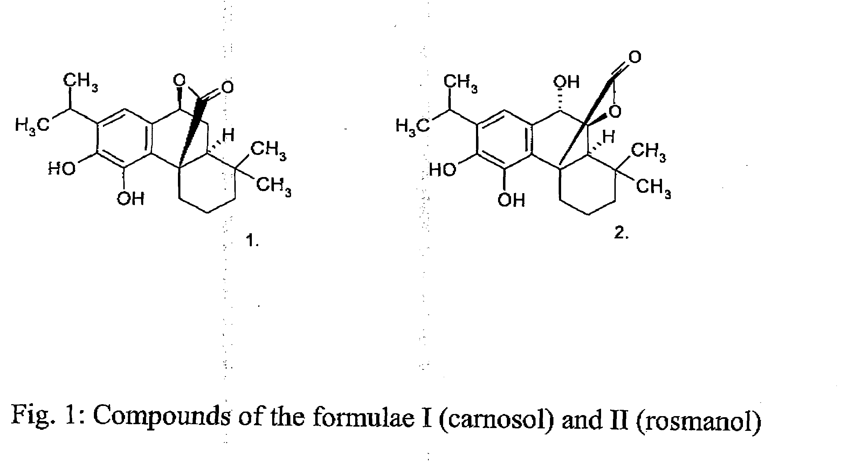

## Claims

1. A dietary or pharmaceutical composition comprising at least camosol and/or rosmanol.

2. The dietary composition according to of claim 1 in form of food such as dairy products (yoghurts), in form of fortified food such as cereal bars and bakery items such as cakes and cookies, in form of dietary supplements such as tablets, pills, granules, dragées, capsules, and effervescent formulations, in form of non-alcoholic drinks such as soft drinks, sport drinks, fruit juices, lemonades, near-water drinks, teas and milk based drinks, in form of liquid food such as soups and dairy products (muesli drinks).

3. Use of the dietary composition according to claim 1 or claim2 as antidepressant, mood/vitality improver, stress reliever, condition improver, reducer of anxiety, reducer of obsessive-compulsive behaviour, relaxant, sleep improver and/or insomnia alleviator.

4. Carnosol or rosmanol of formulae I and II as defined in claim 1 or claim 3 for use as medicament.

5. Carnosol or rosmanol of formulae I and II as defined in claim 1 for use as medicament in the treatment of a disorder connected to impaired neurotransmission.

6. Carnosol or rosmanol of formulae I and II as defined in claim 1 for use according to claim 4 as antidepressant, mood/vitality improver, stress reliever, condition improver, reducer of anxiety, reducer of obsessive-compulsive behaviour, relaxant, sleep improver and/or insomnia alleviator.

7. Use of carnosol or rosmanol thereof of formulae I and II as defined in claim. 1 for the manufacture of a composition for the treatment of a disorder connected to impaired neurotransmission.

8. The use according to claim 7, wherein the composition is an antidepressant, a mood/vitality improver, a stress reliever, a condition improver, a reducer of anxiety, a reducer of obsessive-compulsive behaviour, a relaxant, a sleep improver and/or an insomnia alleviator.

9. A method for the treatment of a disorder connected to impaired neurotransmission in animals including humans, said method comprising administering an effective dose of carnosol or rosmanol of formulae I and II as defined in claim 1 to animals including humans which are in need thereof.

10. The method according to claim 9, wherein the animal is a human, a pet animal or a farm animal.
